# EUROPÄISCHE PATENTSCHRIFT

(11) **EP 1 224 166 B1**
(45) Veröffentlichungstag und Bekanntmachung des Hinweises auf die Patenterteilung: **17.09.2003**
(21) Anmeldenummer: 00972725.6
(22) Anmeldetag: 11.10.2000
(51) Int. Cl.: C07C 315/00, C07C 315/04, C07C 317/44

(54) **VERFAHREN ZUR HERSTELLUNG VON N-(4,5-BIS-METHANSULFONYL-2-METHYL-BENZOYL)-GUANIDIN, HYDROCHLORID**
METHOD OF PRODUCING N-(4,5-BIS-METHANESULFONYL-2-METHYL-BENZOYL)-GUANIDINE, THE HYDROCHLORIDE THEREOF
PROCEDE DE FABRICATION DE CHLORHYDRATE DE N-(4,5-BISMETHANESULFONYL-2-METHYL-BENZOYL)-GUANIDINE

(30) Priorität: 26.10.1999 DE 19951418
(43) Veröffentlichungstag der Anmeldung: 24.07.2002
(73) Patentinhaber: MERCK PATENT GmbH, 64271 Darmstadt (DE)
(72) Erfinder: GERICKE, Rolf, 64342 Seeheim (DE); BAUMGARTH, Manfred, 64297 Darmstadt (DE); BENDER, Hans, Markus, 83104 Ostermünchen (DE); LADSTETTER, Bernhard, 83104 Ostermünchen (DE)
(86) Internationale Anmeldenummer: EP0009977
(87) Internationale Veröffentlichungsnummer: WO01030750

(56) Entgegenhaltungen:
- EP-A- 0 758 644
- A. ULMAN, ET AL.: "Novel synthesis of 4-[alkyl(aryl)sulphonyl]benzaldehydes: alkyl(aryl)sulphinate anion as a nucleophile in aromatic substitutions" JOURNAL OF ORGANIC CHEMISTRY, Bd. 54, Nr. 19, 15. September 1989 (1989-09-15), Seiten 4691-4692, XP002160704 American Chemical Society, Washington, DC, US ISSN: 0022-3263 in der Anmeldung erwähnt
- M. BAUMGARTH, ET AL.: "(2-Methyl-5- (methylsulphonyl)benzoyl)guanidine Na+/H+ antiporter inhibitors" JOURNAL OF MEDICINAL CHEMISTRY, Bd. 40, Nr. 13, 20. Juni 1997 (1997-06-20), Seiten 2017-2034, XP000907364 American Chemical Society, Washington, DC, US ISSN: 0022-2623

## Beschreibung

Die vorliegende Erfindung betrifft N-(4,5-Bis-methansulfonyl-2-methylbenzoyl)-guanidin, Hydrochlorid Hydrat sowie ein Verfahren zur Herstellung von N-(4,5-Bis-methansulfonyl-2-methyl-benzoyl)-guanidin, Hydrochlorid sowie des Hydrochlorid Hydrats. N-(4,5-Bis-methansulfonyl-2-methylbenzoyl)-guanidin, Hydrochlorid Hydrat ist ein NHE-1 selektiver Na⁺/H⁺ - Antiporter Hemmstoff.

Sulfonyl-benzoylguanidine sind bekannt und beispielsweise beschrieben in EP 0 758 644 A1. Bei diesen Substanzen handelt es sich um Inhibitoren des zellulären Na⁺/H⁺-Antiporters, d.h. um Wirkstoffe, die den Na⁺/H⁺-Austauschmechanismus der Zellen hemmen (Düsing et al., Med. Klin. 1992, 87, 367-384,) und die somit gute Antiarrhythmika darstellen, die sich insbesondere zur Behandlung von Arrhythmien eignen, die als Folge von Sauerstoffmangel auftreten.

Diese Substanzen zeigen eine gute kardioprotektive Wirkung und eignen sich daher besonders zur Behandlung des akuten Myokardinfarkts, der Infarkt-Prophylaxe, Post-Infarktbehandlung, chronischer Herzinsuffizienz und zur Behandlung von Angina pectoris. Ferner wirken sie allen pathologischen hypoxischen und ischämischen Schädigungen entgegen, so dass die dadurch primär oder sekundär verursachten Krankheiten behandelt werden können. Diese Wirkstoffe sind ebenfalls für präventive Anwendungen gut geeignet.

Aufgrund der protektiven Wirkung dieser Substanzen bei pathologischen hypoxischen oder ischämischen Situationen resultieren daraus weitere Anwendungsmöglichkeiten bei chirurgischen Eingriffen zum Schutz zeitweilig minderversorgter Organe, bei Organtransplantationen zu Schutz der entnommenen Organe, bei angioplastischen Gefäß- oder Herzeingriffen, bei Ischämien des Nervensystems, bei der Therapie von Schockzuständen und zur Verhinderung der essentiellen Hypertonie.

Ferner können diese Verbindungen auch als Therapeutika bei durch Zellproliferation bedingten Erkrankungen wie Arteriosklerose, Diabetes und diabetischen Spätkomplikationen, Tumorerkrankungen, fibrotischen Erkrankungen, insbesondere von Lunge, Leber und Nieren sowie Organhypertrophien und -hyperplasien, eingesetzt werden. Darüber hinaus eignen sich die Verbindungen zur diagnostischen Anwendung zur Erkennung von Krankheiten, die von einer gesteigerten Aktivität des Na⁺/H⁺-Antiporters z.B. in Erythrozyten, Thrombozyten oder Leukozyten begleitet werden.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe Verwendung finden.

Der Erfindung lag die Aufgabe zugrunde, eine hochwirksame Verbindung mit einer sehr guten oralen Resorptionseigenschaft aufzufinden.

Als hervorragend geeignete und hochwirksame Substanz hat sich das N-(4,5-Bis-methansulfonyl-2-methyl-benzoyl)-guanidin, Hydrochlorid Hydrat herauskristallisiert, die sich durch eine besonders gute orale Resorptionseigenschaft auszeichnet. N-(4,5-Bis-methansulfonyl-2-methylbenzoyl)-guanidin, Hydrochlorid Hydrat wird daher bevorzugt in oraler Form verabreicht.

Die Resorption aus dem Verdauungstrakt nach oraler Verabreichung läßt sich durch Vergleich der im Blutplasma bestimmten Konzentrationen des verabreichten Wirkstoffs nach oraler und intravenöser Verabreichung (dosisnormalisierte AUCₚₒ / AUCᵢᵥ) [AUC = area under the curve] berechnen. Die Ratte zeigte eine Resorptionsquote von 98% (der oral verabreichten radioaktiv markierten Susbtanz). Beim Hund wurden Bioverfügbarkeiten des Hydrochlorid Hydrats von 88% bis 99%, bei 2 Affen von 75% und 96% gefunden. Da die Resorptionsquote mindestens gleich oder größer der bestimmten Bioverfügbarkeit ist, wurde also auch bei diesen Tierarten eine sehr gute Resorption nachgewiesen

Gegenstand der Erfindung ist daher N-(4,5-Bis-methansulfonyl-2-methylbenzoyl)-guanidin, Hydrochlorid Hydrat. Diese Erfindung ist als Auswahlerfindung zu EP 0 758 644 anzusehen.

Da diese Substanz sehr vielversprechend ist, ist ihre Herstellung von größtem Interesse. Die Darstellung der freien N-(4,5-Bis-methansulfonyl-2-methyl-benzoyl)-guanidin Base und ihren Analoga ist in der bereits zitierten EP 0 758 644 A1 beschrieben worden.

Die bekannten Synthesen umfassen jedoch eine Vielzahl von Einzelschritten mit zum Teil unbefriedigenden Ausbeuten und auch mit umweltbelastenden und gefährlichen Reaktionsbedingungen, wie z.B. die Umsetzung mit Methylmercaptam oder die Oxidation des Thioethers zum Sulfon.

Es besteht daher ein großes Interesse, ein verbessertes Herstellungsverfahren für das N-(4,5-Bis-methansulfonyl-2-methyl)-benzoyl)-guanidin, dessen Hydrochlorid und Hydrochlorid Hydrat zu finden.

Aufgabe der vorliegenden Erfindung war daher ebenfalls, eine neue Synthesevariante für den Na⁺/H⁺-Antiporter zu finden, welche kürzer und auch effektiver im Vergleich zu herkömmlichen Methoden ist.

Gegenstand der Erfindung ist ein Verfahren zur Herstellung des Benzoylguanidinderivates N-(4,5-Bis-methansulfonyl-2-methyl)-benzoyl)-guanidin, Hydrochlorid der Formel I, 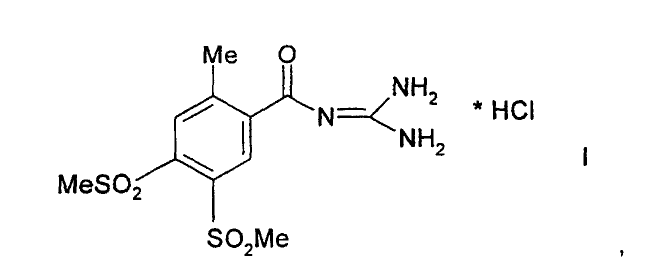 worin Me Methyl bedeutet, sowie des Hydrochlorid Hydrats, welches dadurch gekennzeichnet ist,
dass zunächst durch Umsetzung der Ausgangsverbindung der Formel II 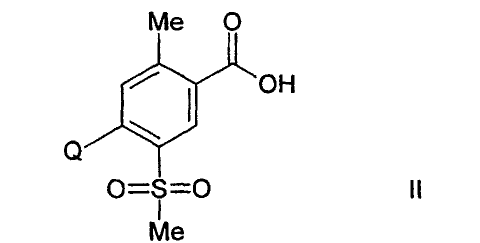 worin
Me eine Methylgruppe und Q Fluor oder Chlor bedeutet,
in einer nucleophilen Substitution am aktivierten Aromaten mit einem Methansulfinat die 4-Methansulfonylgruppe in einer einstufigen Reaktion eingeführt wird,
dann im zweiten Schritt die Verbindung der Formel III 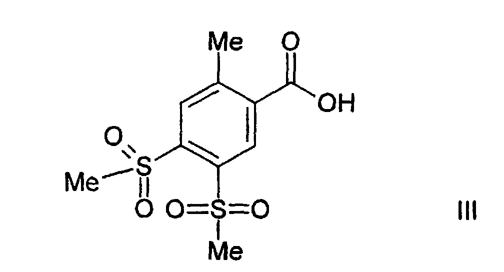 in ein Säurechlorid umwandelt und mit Guanidin zu N-(4,5-Bismethansulfonyl-2-methyl)-benzoyl)-guanidin umsetzt,
und im dritten Schritt durch Umsetzung in wäßriger HCI in das Hydrochlorid der Formel I und/oder dessen Hydrochlorid Hydrat überführt.

Die Herstellung der Ausgangsverbindung der Formel II gelingt beispielsweise ausgehend von 2-Brom-5-chlor-toluol durch Halogen-Metall-Austausch und CO₂-Behandlung zu 4-Chlor-2-methyl-benzoesäure und anschließender Umsetzung von 4-Chlor-2-methyl-benzoesäure mit Chlorsulfonsäure, Natriumsulfit und Methyliodid zu 4-Chlor-2-methyl-5-methylsulfonyl-benzoesäure oder durch Umsetzung von 2-Brom-5-chlortoluol mit Methansulfonsäure und Thionylchlorid in einer Friedel-Craftsartigen Reaktion in Anwesenheit eines Friedel-Crafts-Katalysators zu 4-Chlor-2-methyl-4-methylsulfonyl-phenylbromid und anschließendem Austausch des Broms gegen eine Carboxylgruppe durch eine Palladiumkatalysierte Carbonylierungsreaktion im Autoklaven bei erhöhtem Druck und erhöhter Temperatur zu 4-Chlor-2-methyl-5-methylsulfonylbenzoesäure. Die gewählten Reaktionsbedingungen sind literaturbekannt (Lit.: Houben-Weyl, Methoden der Organ. Chemie, Georg-Thieme-Verlag, Stuttgart). Es können jedoch auch andere, hier nicht näher erläuterte literaturbekannte Verfahren zur Herstellung von Verbindungen der Formel II verwendet werden.

Methansulfinat bedeutet ein Alkalimetallsalz der Methansulfinsäure, insbesondere Natriummethansulfinat oder Kaliummethansulfinat, oder ein Erdalkalimetallsalz der Methansulfinsäure, insbesondere Calciummethansulfinat oder Magnesiummethansulfinat. Besonders bevorzugt wird Natriummethansulfinat eingesetzt.

Die Umsetzung der Verbindung der Formel II mit einem Methansulfinat, bevorzugt Natriummethansulfinat, erfolgt analog zu der Methode von A. Ulman et al., J. Org. Chem. 1989, 54, 4691-4692. Bevorzugt findet die Reaktion in einem polaren Lösungsmittel und bei Reaktionstemperaturen zwischen 10 und 200°, bevorzugt zwischen 50 und 180°, besonders bevorzugt zwischen 80 und 140 ° statt. Besonders bevorzugte Lösungsmittel sind Dimethylsulfoxid (DMSO), N,N-Dimethylformamid (DMF) oder 1-Methyl-2-pyrrolidon (NMP), ganz besonders bevorzugt wird DMF oder NMP verwendet. Das Methansulfinat wird in der Regel im Überschuß eingesetzt. Unter den genannten Reaktionsbedingungen entsteht ausschließlich die 4,5-Bis-methansulfonyl-2-methyl-benzoesäure der Formel III.

Die Guanidierung der Verbindung der Formel III in Schritt 2 ist nicht auf die Säurechloridmethode beschränkt, bei der beispielsweise die Verbindung der Formel III mit Thionylchlorid zum Säurechlorid und weiter mit Guanidin umgesetzt wird. Es gibt eine Vielzahl von literaturbekannten Methoden, die die Einführung einer Guanidinogruppe ermöglichen (z.B. in den Standardwerken wie Houben-Weyl, Methoden der organischen Chemie, Georg-Thieme-Verlag, Stuttgart).
Unter anderem kann für eine Guanidierung in Schritt 2 die freie Säure der Formel III mit N-(Benzyloxy-carbonyl)-guanidin umgesetzt werden, wobei die nachfolgende Abspaltung der Benzyloxycarbonyl-Schutzgruppe (Abkürzung = Z) die Guanidinogruppe freisetzt, wie beschrieben in DE 199 19 349. Zur Herstellung von Benzyloxycarbonyl-guanidin siehe M. Goodman et al., PCT Int. Appl. WO 9852917, 1998, K. Nowak, Rocz. Chem. 1969, 43, 231-232 oder R. Krug und K. Nowak, Rocz. Chem. 1967, 41, 1087-1091). Die Kupplung erfolgt mit Hilfe der bekannten Mukaiyama-Methode, vgl. T. Mukaiyama, Angew. Chem., Int. Ed. Engl. 1979, 18, 707-721. Die Abspaltung der Z-Schutzgruppe durch katalytische Hydrierung kann nach den dafür allgemeinen Bedingungen durchgeführt werden (Lit.: T.W. Greene, P.G.M. Wuts, *Protective Groups in Organic Chemistry*, 2. Aufl., Wiley, New York 1991 oder P.J. Kocienski, *Protecting Groups*, 1. Aufl., Georg Thieme Verlag, Stuttgart - New-York, 1994).

Die in Schritten 1 und 2 erfindungsgemäß hergestellte Base N-(4,5-Bismethansulfonyl-2-methyl)-benzoyl)-guanidin kann auch durch andere Säuren als HCI nach literaturbekannten Methoden in ein Salz überführt werden.Die dafür in Frage kommenden Säuren sind in EP 0 785 644 genannt.

Bei der in EP 0 758 644 beschriebenen Methode zur Darstellung von Sulfonyl-benzoylguanidinen wird die zur Carboxylgruppe para-ständige Sulfonylgruppe über einen nucleophilen Halogen-Schwefelalkyl-Austausch nebst anschließender Oxidation des erhaltenen Thiophenolethers eingeführt.

Das nun in dieser Erfindung vorliegende Verfahren führt die para-ständige Sulfonylgruppe in einem einstufigen Reaktionsschritt ein. Die Zahl der Syntheseschritte und die damit verbundenen Herstellkosten werden verringert. Weiterhin entfällt die Umsetzung mit Methylmercaptan sowie die Oxidation, die im großtechnischen Maßstab durch die Möglichkeit der Persäure-Bildung besondere technische Sicherheitsmaßnahmen erforderlich macht.

Somit steht ein effektives Verfahren zur Herstellung von N-(4,5-Bismethansulfonyl-2-methyl -benzoyl)-guanidin, Hydrochlorid sowie dessen Hydrochlorid Hydrat zur Verfügung, welches wesentlich verbessert zum bisher bekannten Verfahren ist, sowohl in Bezug auf die Anzahl der Syntheseschritte als auch bezüglich der Gesamtausbeute.

Alle vorstehenden und nachfolgenden Temperaturangaben werden in °C angegeben.

### Beispiel:

### 1. Synthese von 4,5-Bis-methansulfonyl-2-methyl-benzoesäure

6 kg 4-Chlor-2-methyl-5-(methylsulfonyl)benzoesäure werden bei Raumtemperatur (25°) in 15 I N,N-Dimethylformamid (DMF) gelöst und anschließend auf 50° erwärmt. Zu dieser Lösung werden 3,6 kg Natriummethansulfinat zugegeben. Die Innentemperatur wird dauraufhin auf 120° erhöht und 2 Tage bei dieser Temperatur gerührt, wobei nach 24 h noch einmal 3 kg Natriummethansulfinat zugegeben werden.
Nach Erkalten auf 25° wird die Reaktionslösung in 40 I Wasser eingetragen und 300 g Aktivkohle und 1 kg Kieselgur zugesetzt. Das Filtrat wird mit 5 I Eis versetzt und 3,5 I konzentrierte Chlorwasserstoffsäure zugetropft (pH = 1). Man erhält nach Umkristallisation aus 2-Propanol 3,8 kg 4,5-Bismethansulfonyl-2-methyl-benzoesäure; Fp. 234-235°.

### 2. Synthese von N-(4,5-Bis-methansulfonyl-2-methyl-benzoyl)-guanidin

### 2.1. Synthese des Säurechlorids

15 I Thionylchlorid werden bei 40° vorgelegt und mit 50 ml DMF versetzt. Unter Rühren werden 3,8 kg 4,5-Bis-methansulfonyl-2-methyl-benzoesäure langsam eingetragen und dann 1 h bei Siedehitze gerührt. Nach Abkühlen wird das überschüssige SOCl₂ abezogen und der Rückstand mehrmals mit 5 I Toluol codestilliert. Man erhält 4,5-Bis-methansulfonyl-2-methylbenzoylchlorid, welches als Rohprodukt weiter umgesetzt wird.

### 2.2. Synthese des Guanidins

1,4 kg Natrium werden unter Schutzgas in 15 l siedendem Methanol gelöst und mit weiteren 10 I Methanol verdünnt. Zu der auf 20-22° gekühlten Lösung werden 5,9 kg Guanidiniumhydrochlorid zugegeben und 1 h gerührt. Danach wird das entstandene Natriumchlorid abfiltriert und die Lösung eingeengt. Der Rückstand wird mit Toluol codestilliert und dann in 10 I DMF aufgenommen.

### 2.3 Synthese von N-(4,5-Bis-methansulfonyl-2-methyl-benzoyl)-guanidin

Zu der nach 2.2. hergestellten Guanidinlösung wird bei 12° eine Lösung des 4,5-Bis-methansulfonyl-2-methyl-benzoylchlorids, hergestellt nach 2.1., in 5 I DMF zugetropft. Das Reaktionsgemisch wird 5 h bei 20° gerührt und langsam mit 45 I kaltem Wasser (0-5°) versetzt. Die ausgeschiedenen Kristalle werden abfiltriert und mit Eiswasser, Acetonitril und Diethylether nachgewaschen. Die Rohkristalle werden in 315 I Acetonitril/Wasser (20:1) heiß gelöst. Die Lösung wird mit 200 g Aktivkohle behandelt, filtriert und auf 0° abgekühlt.
Man erhält N-(4,5-Bis-methansulfonyl-2-methyl-benzoyl)-guanidin als Acetonitriladdukt in einer Ausbeute von 64,8%; Fp. 233-234°.

### 3.1. Synthese von N-(4,5-Bis-methansulfonyl-2-methyl-benzoyl)-guanidin, Hydrochlorid Hydrat

2,7 kg N-(4,5-Bis-methansulfonyl-2-methyl-benzoyl)-guanidin werden bei 60° in 25 I Wasser suspendiert und 10,6 l 1N HCI-Lösung zugegeben. Bei Erwärmen auf 80° erhält man eine klare Lösung. Die Lösung läßt man langsam abkühlen, wobei die Kristallisation bei 50° beginnt. Man erhält N-(4,5-Bis-methansulfonyl-2-methyl-benzoyl)-guanidin, Hydrochlorid Hydrat in einer Ausbeute von 97%, Fp. 181-188°.

### 3.2. Synthese von N-(4,5-Bis-methansulfonyl-2-methyl-benzoyl)-guanidin, Hydrochlorid

Das aus 3.1. erhaltene N-(4,5-Bis-methansulfonyl-2-methyl-benzoyl)-guanidin, Hydrochlorid Hydrat wird bei 120° im Vakuum bis zur Gewichtskonstanz getrocknet. Man erhält N-(4,5-Bis-methansulfonyl-2-methyl-benzoyl)-guanidin, Hydrochlorid.

### 3.3. Synthese von N-(4,5-Bis-methansulfonyl-2-methyl-benzoyl)-guanidin, Hydrochlorid

2,7 kg N-(4,5-Bis-methansulfonyl-2-methyl-benzoyl)-guanidin werden bei 60° in 25 I Ethanol suspendiert und 10,6 I 1N HCI-Lösung zugegeben. Bei Erwärmen auf 80° erhält man eine klare Lösung. Die Lösung läßt man langsam abkühlen, wobei die Kristallisation bei 50° beginnt. Das erhaltene N-(4,5-Bis-methansulfonyl-2-methyl-benzoyl)-guanidin, Hydrochlorid wird anschließend bei 60° bis zur Gewichtskonstanz getrocknet. Man erhält N-(4,5-Bis-methansulfonyl-2-methyl-benzoyl)-guanidin, Hydrochlorid.

## Patentansprüche

1. N-(4,5-Bis-methansulfonyl-2-methyl-benzoyl)-guanidin, Hydrochlorid Hydrat.

2. Verfahren zur Herstellung von N-(4,5-Bis-methansulfonyl-2-methylbenzoyl)-guanidin, Hydrochlorid der Formel I, 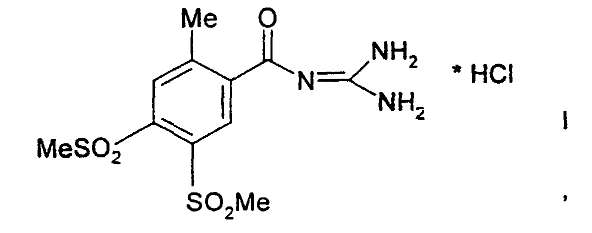 worin Me Methyl bedeutet, sowie des Hydrochlorid Hydrats, welches **dadurch gekennzeichnet ist,**
**dass** zunächst durch Umsetzung der Ausgangsverbindung der Formel II 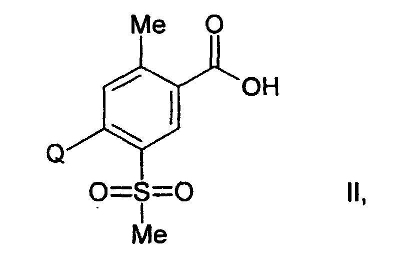 worin
Me eine Methylgruppe und Q Fluor oder Chlor bedeutet,
in einer nucleophilen Substitution mit einem Methansulfinat die 4-Methansulfonylgruppe in einer einstufigen Reaktion eingeführt wird,
dann im zweiten Schritt die Verbindung der Formel III 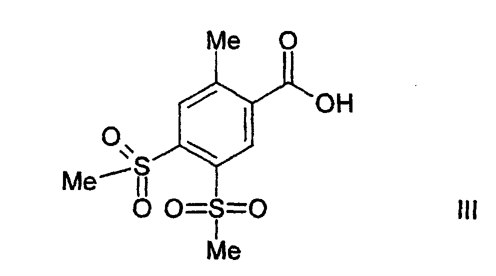 in das Säurechlorid umgewandelt und mit Guanidin zu N-(4,5-Bismethansulfonyl-2-methyl)-benzoyl)-guanidin umgesetzt wird,
und im dritten Schritt durch Umsetzung in wäßriger HCI in das Hydrochlorid der Formel I oder das Hydrochlorid Hydrat überführt wird.

3. Verfahren nach Anspruch 2, **dadurch gekennzeichnet, dass** im ersten Schritt Natriummethansulfinat verwendet wird.

4. Verfahren nach Anspruch 2 oder 3, **dadurch gekennzeichnet, dass** im ersten Schritt ein polares Lösungsmittel verwendet wird.

5. Verfahren nach Anspruch 2 bis 4, **dadurch gekennzeichnet, dass** im ersten Schritt eine Reaktionstemperatur zwischen 80 und 140° eingestellt wird.

## Claims

1. N-(4,5-Bismethanesulfonyl-2-methylbenzoyl)guanidine, hydrochloride hydrate.

2. Process for the preparation of N-(4,5-bismethanesulfonyl-2-methyl-benzoyl)guanidine, hydrochloride, of the formula I 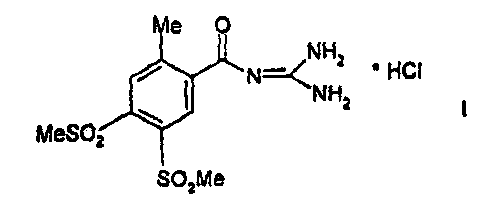 in which Me denotes methyl, and of the hydrochloride hydrate, which is **characterised in that**
firstly the 4-methanesulfonyl group is introduced in a one-step reaction by reaction of the starting compound of the formula II 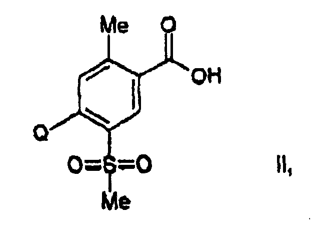 in which
Me is a methyl group and Q is fluorine or chlorine,
in a nucleophilic substitution with a methanesulfinate,
then in a second step the compound of the formula III 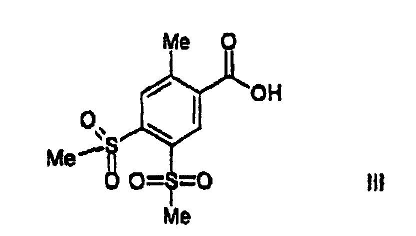 is converted into the acid chloride and reacted with guanidine to give N-(4,5-bismethanesulfonyl-2-methylbenzoyl)guanidine,
and in the third step is converted into the hydrochloride of the formula I or the hydrochloride hydrate by reaction in aqueous HCl.

3. Process according to Claim 2, **characterised in that** sodium methane sulfinate is used in the first step.

4. Process according to Claim 2 or 3, **characterised in that** a polar solvent is used in the first step.

5. Process according to Claims 2 to 4, **characterised in that** a reaction temperature between 80 and 140° is set in the first step.

## Revendications

1. Chlorhydrate hydraté de N-(4,5-bisméthanesulfonyl-2-méthylbenzoyl)-guanidine.

2. Procédé de préparation du chlorhydrate de N-(4,5-bisméthane-sulfonyl-2-méthylbenzoyl)guanidine de formule I 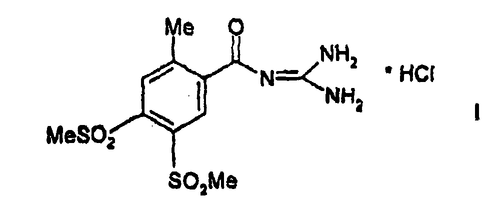 dans laquelle Me désigne méthyle, et du chlorhydrate hydraté, qui est **caractérisé en ce que**
d'abord, le groupement 4-méthanesulfonyle est introduit dans une réaction à une étape par la réaction du composé de départ de formule II 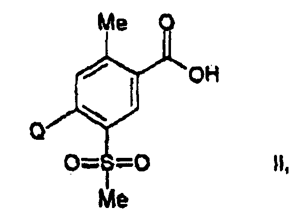 dans laquelle
Me désigne un groupement méthyle et Q désigne fluor ou chlore,
dans une substitution nucléophile avec un méthanesulfinate,
puis, dans une deuxième étape, le composé de formule III 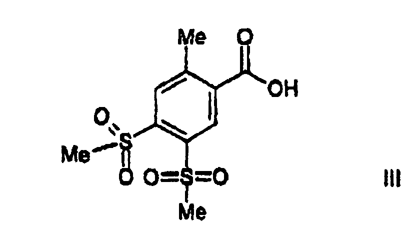 est converti en chlorure d'acide et réagi avec la guanidine pour donner la N-(4,5-bisméthanesulfonyl-2-méthylbenzoyl)guanidine,
et dans la troisième étape, est converti en chlorhydrate de formule I ou le chlorhydrate hydraté par une réaction dans l'HCl aqueux.

3. Procédé selon la revendication 2, **caractérisé en ce que** le méthanesulfinate de sodium est utilisé dans la première étape.

4. Procédé selon la revendication 2 ou 3, **caractérisé en ce qu'**un solvant polaire est utilisé dans la première étape.

5. Procédé selon les revendications 2 à 4, **caractérisé en ce qu'**une température de réaction comprise entre 80 et 140° est consignée dans la première étape.
